# EUROPÄISCHE PATENTANMELDUNG

(11) **EP 2 308 998 A1**
(43) Veröffentlichungstag der Anmeldung: **13.04.2011**
(21) Anmeldenummer: 09011941.3
(22) Anmeldetag: 18.09.2009
(51) Int. Cl.: C12Q 1/68

(54) **Verfahren zur frühen Diagnose von Karzinomen des Anogenitaltraktes**

(71) Anmelder: Universitätsklinikum Jena Körperschaft des öffentlichen Rechts und Teilkörperschaft der Friedrich-Schiller-Universität Jena, 07743 Jena (DE)
(72) Erfinder: Dürst, Matthias, Prof. Dr., 07743 Jena (DE); Hansel, Alfred, Dr., 07745 Jena (DE); Steinbach, Daniel, 08115 Lichtentanne (DE)
(74) Vertreter: Schüssler, Andrea

(57) **Zusammenfassung**

Beschrieben wird ein Verfahren zur frühen Diagnose von Karzinomen des Anogenitaltraktes, vorzugsweise des Zervixkarzinoms, sowie deren Vorstufen. Das Verfahren basiert auf der Bestimmung des Methylierungsstatus von ASTN1 (Astrotactin 1), DLX1 (distal-less homeobox 1), EDIL3 (EGFlike repeats and discoidin I-like domains 3), ITGA4 (α4-Integrin), RXFP3 (relaxin/insulin-like family peptide receptor 3)und/oder ZNF671 (Zinkfinger-Protein 671). Eine DNA-Methylierung im Promotorbereich und/oder der 5'-Region dieser Gene weist auf das Vorliegen eines Karzinoms des Anogenitaltraktes oder dessen schwergradige Vorstufen hin. Außerdem werden Kits beschrieben, die die Durchführung des erfindungsgemäßen Diagnoseverfahrens gestatten.

## Beschreibung

Die vorliegende Erfindung betrifft ein Verfahren zur frühen Diagnose von Karzinomen des Anogenitaltraktes, vorzugsweise des Zervixkarzinoms, sowie deren Vorstufen. Das Verfahren basiert auf der Bestimmung des Methylierungsstatus von ASTN1 (Astrotactin 1), DLX1 (distal-less homeobox 1, Transkriptionsfaktor), EDIL3 (EGF-like repeats and discoidin I-like domains 3), ITGA4 (α4-Integrin), RXFP3 (relaxin/insulin-like family peptide receptor 3) und/oder ZNF671 (Zinkfinger-Protein 671, Tranksriptionsfaktor). Eine DNA-Methylierung in Guanin/Cytosin-reichen Bereichen, den sogenannten CpG-Inseln, im Promotorbereich und/oder der 5'-Region wenigstens eines dieser Gene ist charakteristisch für Karzinome oder Karzinomvorstufen des Anogenitaltraktes. Der Nachweis entsprechend veränderter DNA wird diagnostisch genutzt. Die vorliegende Erfindung betrifft außerdem Kits, die die Durchführung des erfindungsgemäßen Diagnoseverfahrens gestatten.

Gebärmutterhalskrebs (Zervixkarzinom) ist die zweithäufigste bösartige Krebserkrankung bei Frauen weltweit. Er entwickelt sich im Zuge einer Infektion mit sog. Hochrisiko-humanen-Papillomviren (hr-HPV) über Vorstufen, die als zervikale intraepitheliale Neoplasien (CIN) bezeichnet werden. CIN1: (leichte Dysplasie) geht von basal aus bis höchstens einem Drittel der Höhe des Epithels; CIN2: (mittelgradige Dysplasie) bis zu zwei Drittel des Epithels; CIN3: (hochgradige Dysplasie) durchzieht fast das gesamte Epithel. CIN2 und CIN3 werden als schwergradige Dysplasien bezeichnet. Letztere haben ein hohes Risiko in ein Zervixkarzinom überzugehen. In der vorliegenden Anmeldung werden CIN2, CIN3 und Zervixkarzinom unter dem Begriff CIN2+ zusammengefasst. Neben einer hr-HPV Infektion sind auch weitere Faktoren an der Zervixkarzinogenese beteiligt.

Der bestehende Vorsorgetest für den Nachweis eines Zervixkarzinoms und dessen Vorstufen (CIN) beruht auf einem zytomorphologischen Verfahren (Pap-Test). Der Pap-Test ist höchst anfällig für Fehler, da wenige Krebs- oder CINverdächtige Zellen in einem Hintergrund tausender normaler Zellen mikroskopisch erkannt werden müssen. Des Weiteren ist die Beurteilung der Zellmorphologie äußerst subjektiv. Diese Schwächen führen dazu, dass die Sensitivität des Pap-Tests für den Nachweis von CIN2+ 53% und die Spezifität 96.3% beträgt (Cuzick et al., 2006; Int J Cancer, 119:1095-1101).

Durch molekulare Testverfahren konnte die Krebsvorsorge wesentlich verbessert werden. Da bis auf wenige Ausnahmen alle Zervixkarzinome und deren Vorstufen hr-HPV-DNA enthalten, erscheint HPV-DNA als idealer Marker für die Krebsvorsorge. In verschiedenen veröffentlichten Studien konnte gezeigt werden, dass der HPV-DNA-Test (ein PCR-Verfahren) für den Nachweis von CIN2+ eine Sensitivität von 96.1% und eine Spezifität von 90.7% aufweist. Allerdings ist der positive Vorhersagewert eines HPV-DNA-Tests für CIN2+ mit nur 20.3% erwartungsgemäß schlecht, da viele Frauen lediglich mit HPV infiziert sind, aber keine Krebsvorstufen aufweisen (Cuzick et al., 2006; Int J Cancer, 119:1095-1101). Somit besteht ein Bedarf an einer verbesserten Diagnostik von Karzinomen des Anogenitaltraktes, insbesondere des Zervixkarzinoms.

Der vorliegenden Erfindung liegt somit die Aufgabe zugrunde, ein Verfahren bereitzustellen, mit dem Karzinome des Anogenitaltraktes wie das Zervixkarzinom und vor allem deren Vorstufen frühzeitig und zuverlässig diagnostiziert werden können.

Erfindungsgemäß wird dies durch die Gegenstände in den Patentansprüchen erreicht.

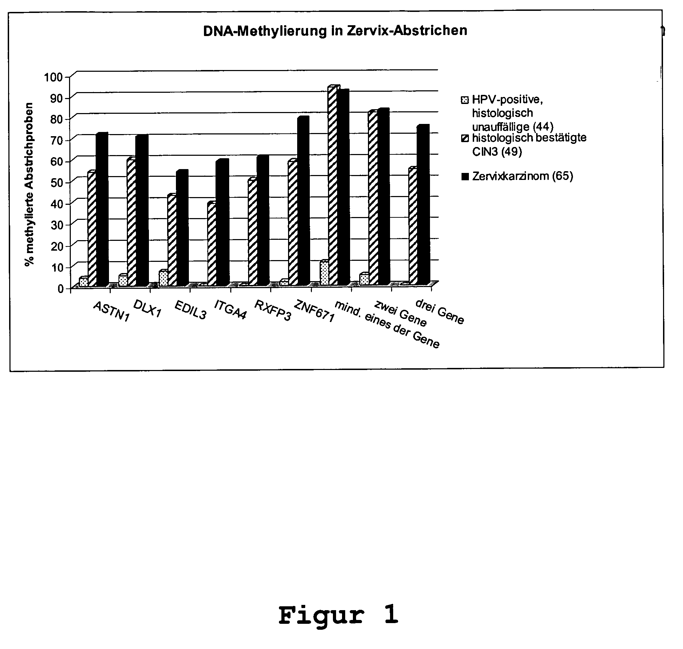

Die vorliegende Erfindung beruht auf den nachfolgend näher beschriebenen Erkenntnissen des Anmelders hinsichtlich des Zusammenhangs zwischen dem Methylierungsstatus bestimmter Gene und der Entwicklung von Karzinomen des Anogenitaltraktes. In Karzinomen und deren Vorstufen werden im Vergleich zum entsprechenden Normalgewebe die Cytosin/Guanin-reichen CpG-Inseln im Upstream-, Promotor- und in promoternahen Exonbereichen von Genen häufig methyliert. Diese Methylierung von Genen geschieht nicht willkürlich, sondern ist von der jeweiligen Tumor-Entität abhängig (Esteller, 2007, Hum. Mol. Genet., 16: R50-R59). Im Rahmen der Erfindung wurden daher Analysen zur Methylierung durchgeführt, mit dem Ziel, Gene zu identifizieren, die besonders häufig in DNA aus Abstrichen von Patientinnen mit histopathologisch bestätigter CIN3 sowie von Patientinnen mit Zervixkarzinom methyliert waren. Des Weiteren sollten die Gene in DNA aus Zervixabstrichen von zytologisch unauffälligen, aber hr-HPV-positiven Frauen nur sehr selten bis gar nicht methyliert sein. Unter mehr als 100 Kandidatengenen erfüllten nur 6 diese Bedingungen. Die hier aufgeführte Analyse zeigt erstmals, dass die Methylierung bestimmter Bereiche der Gene ASTN1, DLX1, EDIL3, ITGA4, RXFP3 und ZNF671 einen wertvollen Marker zur Erkennung von Karzinomen des Anogenitaltraktes (bevorzugt: CIN2+) in einer Probe darstellt (siehe Abbildung 1). Die vorliegende Erfindung stellt daher verbesserte Methoden zum Nachweis eines Zervixkarzinoms und dessen schwergradige Vorstufen (CIN2+) in einer Probe (z.B. Zellabstrich des Gebärmutterhalses) bereit.

Die vorliegende Erfindung basiert auf dem (direkten oder indirekten) Nachweis des Methylierungsstatus von ASTN1, DLX1, EDIL3, ITGA4, RXFP3 und ZNF671. Vorzugsweise wird nachgewiesen, ob die Promotorbereiche eines oder mehrerer der Gene ASTN1, DLX1, EDIL3, ITGA4, RXFP3 und ZNF671 methyliert sind. Da Methylierung meist an Promotorregionen von Genen stattfindet, konzentrieren sich Methoden zum Nachweis der Methylierung der relevanten Gene meist auf diese Regionen. Allerdings können Gene auch in anderen als der Promotorregion methyliert sein, da die GC-reichen CpG-Inseln sich nicht nur dort befinden. Der Nachweis einer Methylierung in solchen Bereichen kann auch von diagnostischem Nutzen sein und ist damit auch Gegenstand der vorliegenden Erfindung.

In der vorliegenden Erfindung wird der Methylierungsstatus der Gene ASTN1, DLX1, EDIL3, ITGA4, RXFP3 und ZNF671 vorzugsweise in den Promotorregionen nachgewiesen. Dabei wird untersucht, ob bestimmte Cytosine zu 5-Methylcytosin modifiziert, also methyliert sind oder nicht. In DNA aus einer Probe von einer Frau mit einer hr-HPV Infektion ohne nachweisbare klinische Veränderungen ist die Methylierung in denentsprechenden Positionen der DNA selten bis gar nicht nachweisbar. Bei Frauen mit CIN2+ ist die Wahrscheinlichkeit für eine Methylierung an den gewählten Cytosinresten dagegen hoch.

Der spezifische Nachweis der Methylierung von Genen, die während der Karzinogenese im Vergleich zum Normalgewebe auftritt, kann somit den Pap-Test und den HPV-DNA-Nachweis ergänzen bzw. u.U. ersetzen. Die Spezifität des HPV-Tests für den Nachweis von CIN2+ kann durch parallel durchgeführte Methylierungsanalysen erheblich verbessert werden (siehe Abbildung 1). Als Ausgangsmaterial für den Pap-Test, den HPV-Nachweis und die DNA-Methylierung dient jeweils das gleiche Abstrichmaterial aus den zu testenden Personen.

### Kurze Beschreibung der Figuren

Figur 1: DNA-Methylierung in verschiedenen Abstrichen Graphische Darstellung der Methylierungshäufigkeit der Gene ASTN1, DLX1, EDIL3, ITGA4, RXFP3 und ZNF671 sowie die Häufigkeit der Methylierung jeweils wenigstens eines der Gene in DNA aus Abstrichen von HPV-positiven unauffälligen Frauen (n=44), Patientinnen mit histopathologisch bestätigter CIN3 (n=49) und Patientinnen mit Zervixkarzinom (n=65).

Somit betrifft die vorliegende Erfindung ein Verfahren zum Nachweis von Anogenitalkrebs oder Vorstufen davon in einer Probe, umfassend die Bestimmung des Methylierungsstatus von ASTN1 (Astrotactin 1), DLX1 (distal-less homeoboxl), EDIL3 (EGF-like repeats and discoidin I-like domains 3), ITGA4 (α4-Integrin), RXFP3 (relaxin/insulin-like family peptide receptor 3), und/oder ZNF671 (Zinkfinger-Protein 671), wobei eine nachweisbare Methylierung im Vergleich zu einer Probe von einer gesunden Person ein Hinweis auf Anogenitalkrebs oder einer Vorstufe davon ist.

ASTN1 (Astrotactin 1; accession number NM_004319.1, NM_207108 enthalten in NC_000001.9) ist ein Adhäsionsprotein, das eine wichtige Rolle bei der Migration von neuronalen Zellen spielt. DLX1 (distal-less homeobox1; accession numbers NM_178120, NM_001038493, enthalten in NC_000002.11) ist ein Transkriptionsfaktor und beeinflusst Zelldifferenzierung. EDIL3 (EGF-like repeats and discoidin I-like domains 3; accession number NM_005711, enthalten in NC_000005.8) ist ein Integrin-Ligand und spielt eine wesentliche Rolle bei der Angiogenese. Er könnte wichtig sein bei der Gefäßwandumstrukturierung und -entwicklung. Zudem fördert EDIL3 die Adhäsion von Endothelzellen. ITGA4 (α-4-Integrin; accession number NM_000885, enthalten in NC_000002.10) gehört zur Familie der α-Integrine, welche zusammen mit jeweils einem β-Integrin als integrale Membranproteine auftreten. Sie dienen als Rezeptoren für Fibronectin und spielen somit eine wesentliche Rolle bei der Zelladhäsion. RXFP3 (relaxin/insulin-like family peptide receptor 3; accession number NM_016568, enthalten in NC_000005.8) dient als G-Protein-gekoppelter Rezeptor für Relaxin 3. ZNF671 (accession number NM_024883, enthalten in NC_000019.9) ist ein Transkriptionsfaktor mit einem typischen Zinkfinger-Motiv.

Der hier verwendete Ausdruck "Anogenitalkrebs" umfasst alle Krebsarten des Anogenitaltrakts, also im genitalen, perinealen, analen, und perianalen Bereich.

Der hier verwendete Ausdruck "Vorstufen" bezieht sich auf schwergradige Vorstufen und umfasst CIN2 und CIN3.

Der hier verwendete Ausdruck "Probe" umfasst jegliche Körperproben, in denen DNA-Methylierung nachgewiesen werden kann. Beispiele solcher Körperproben sind Blut, Abstriche, Sputum, Urin, Stuhl, Liquor, Galle, gastrointestinale Sekrete, Lymphflüssigkeit, Knochenmark, Organpunktate und Biopsien. Insbesondere sind Abstriche und Biopsien angesagt, wenn es sich um den Nachweis von Anogenital-Karzinomen, z. B. Zervixkarzinom, handelt. Der Fachmann kennt geeignete Verfahren und Hilfsmittel zur Probeentnahme. Der Fachmann kennt auch Verfahren und Reagenzien zur DNA-Isolierung aus der Probe, z.B. Extraktion mit Phenol/Chloroform oder mittels kommerzieller Kits.

Der hier verwendete Ausdruck "Methylierungsstatus" bezieht sich auf die Methylierung der genomischen DNA im Bereich der Primer-Bindungsstellen der betreffenden Gene, vorzugsweise in GC-reichen CpG-Inseln im 5'- und Promotorbereich.

In einer bevorzugten Ausführungsform wird das erfindungsgemäße Diagnoseverfahren zur Diagnose von Gebärmutterhalskrebs (Zervixkarzinom) und dessen Vorstufen (zusammen CIN2+) verwendet.

In einer weiteren bevorzugten Ausführungsform des erfindungsgemäßen Verfahrens wird die Methylierung der Gene ASTN1, DLX1, EDIL3, ITGA4, RXFP3 und/oder ZNF671 bestimmt. Der hier verwendete Begriff "Methylierung" ist synonym mit dem in der Molekularbiologie gängigen Begriff Hypermethylierung. Er bezeichnet die vom Normalzustand abweichende Methylierung innerhalb eines allgemein an Guanin und Cytosin und besonders an CG-Dinukleotiden reichen DNA-Abschnittes, einer sog. CpG-Insel.

Vorzugsweise handelt es sich bei der für das erfindungsgemäße Verfahren verwendeten Probe um einen Gebärmutterhalsabstrich, der sehr zuverlässige Ergebnisse liefert.

In einer weiteren bevorzugten Ausführungsform des erfindungsgemäßen Verfahrens wird der Methylierungsstatus des Promotorbereichs sowie des 5'- und der promotornahen Exonbereiche eines Gens bestimmt. Diese Bereiche sind besonders aufschlussreich.

Dem Fachmann sind geeignete Verfahren zur Bestimmung des Methylierungsstatus bekannt. Vorzugsweise wird die Methylierung von DNA nach einer vorangegangenen Modifikation von nicht-methylierten Cytosinen durch die Bisulfit-Methode mittels einer sog. methylierungsspezifischen PCR (MSP) unter Verwendung geeigneter Primerpaare nachgewiesen. Bei der Bisulfit-Methode werden unmethylierte Cytosine in Uracil umgewandelt, methylierte Cytosine (5-Methylcytosin) sind vor dieser Umwandlung geschützt. Uracil weist andere Paarungseigenschaften als Cytosin auf, d.h., es verhält sich wie Thymidin. Die MSP ist eine etablierte Technik zum Nachweis von DNA-Methylierung. Die MSP beruht auf Primern und evtl. Sonden, die eine Unterscheidung zwischen methylierter und unmethylierter DNA erlauben, d.h., die Ausbildung eines Amplifikationsproduktes zeigt eine Methylierung an, entspricht somit einem positiven Befund.

Das Design der Primer für die Detektion des Methylierungsstatus hängt von der Lokalisierung und Sequenz der Methylierungsbereiche von ASTN1, DLX1, EDIL3, ITGA4, RXFP3 und ZNF671 ab. Die in dieser Erfindung verwendbaren methylierungsspezifischen Primer für ASTN1, DLX1, EDIL3, ITGA4, RXFP3 und ZNF671 binden während des Testverfahrens nur dann an die Bisulfit-modifizierte Proben-DNA, wenn diese an bestimmten Cytosinen innerhalb der Primerregion methyliert war. Waren diese Bereiche vor Bisulfit-Behandlung nicht methyliert, binden auch die Primer nicht, und es entsteht kein Reaktionsprodukt. So weist die Anwesenheit eines Reaktionsproduktes auf eine Methylierung des DNA-Bereiches des jeweiligen Gens und damit auf das mögliche Vorliegen von, z.B. im Falle von DNA aus Zervixabstrichen, CIN2+ hin.

Besonders bevorzugt ist ein Real-time PCR Verfahren (QMSP), das nicht nur eine qualitative Erfassung der Methylierung, sondern auch eine Quantifizierung der methylierten DNA-Abschnitte erlaubt. Diese MSP kann in einem fluoreszenzbasierten Realtime-Verfahren durchgeführt werden, bei der die Entstehung des methylierungsspezifischen Produktes durch den Einbau eines fluoreszenten Farbstoffes, z. B. SYBR-Green, verfolgt wird. Mit beiden Verfahren (MSP und QMSP) kann die Methylierung von Markergenen in einem hohen Hintergrund von nicht-methylierter DNA nachgewiesen werden (Shames et al., 2007, Cancer Lett. 251:187-98). PCR-basierte Methoden können auch in Hochdurchsatz-Verfahren angewendet werden und sind daher auch für die Krebsvorsorge besonders geeignet.

Noch mehr bevorzugt ist eine auf der "MethyLight"-Technik basierende QMSP, wobei fluoreszierende Sonden für die jeweiligen auf Methylierung zu untersuchenden Genabschnitte verwendet werden. Die Sonde trägt den Fluoreszenzfarbstoff am 5'-Ende und einen Quencher am 3'-Ende. Sie bindet an das PCR-Reaktionsprodukt zwischen den beiden spezifischen Primern. Fluoreszenter Farbstoff wird freigesetzt, sobald die Sonde nach Bindung an die Zielsequenz durch die 5'-3'-Exonucleaseaktivität der DNA-Polymerase abgebaut wird. Die gemessene Fluoreszenz spiegelt die Menge an gebildetem Produkt wider. Durch den Einsatz mehrerer Oligonukleotide und Sonden in einer sog. Multiplex-Reaktion kann die Anzahl der durchzuführenden Reaktionen für zu untersuchende Proben in diesem Verfahren entsprechend vermindert werden (Shames et al., 2007, Cancer Lett. 251:187-98). Geeignete fluoreszente Farbstoffe und Quencher sind dem Fachmann bekannt, z.B. als Fluorophor FAM, HEX^{™}, NED^{™}, ROX^{™}, Texas Red^{™} usw. und als Quencher TAMRA oder BHQ.

In einer besonders bevorzugten Ausführungsform wird das Verfahren der vorliegenden Erfindung als Multiplex-Experiment durchgeführt. Ein solches Multiplex-Experiment erlaubt den Nachweis von, z.B., CIN2+ durch die Analyse des Methylierungsstatus mehrerer Gene, der bekanntermaßen mit dem Vorliegen von CIN2+ in Zusammenhang steht, in einem einzelnen Ansatz pro Probe. Das Multiplexverfahren bietet einige Vorteile, da der Methylierungsstatus des zu testenden Gensets in einer oder zwei Reaktionen pro Probe ermittelt werden kann. Dies führt zu einer erheblichen Ersparnis von Zeit und Materialkosten. Im Multiplex-Experiment werden die methylierungsspezifischen Primer für bis zu fünf zu testende Gene eingesetzt. Zusätzlich wird für jedes Gen jeweils ein weiteres spezifisches Olgonukleotid, eine sog. Sonde eingesetzt. Diese trägt an einem Ende einen fluoreszenten Farbstoff und ist so modifiziert, dass die Fluoreszenz erst dann auftritt, wenn die Sonde spezifisch an während des Experiments entstehende Reaktionsprodukte bindet. Die Sonden tragen unterschiedliche Fluoreszenzgruppen, so dass alle gleichzeitig detektiert werden können. Das erfindungsgemäße Verfahren kann auch mittels der "Microarray"-Technologie durchgeführt werden.

Dem Fachmann sind weitere Methoden zur Bestimmung des Methylierungsstatus bekannt, z.B. solche, die auf einer direkten Bestimmung der Menge des spezifischen Produktes durch Fluoreszenz basieren. So kann für das erfindungsgemäße Diagnoseverfahren auch die "molecular beacon"-Technologie verwendet werden. Molecular Beacons sind Oligonucleotide, die sowohl mit einem Reporter-Fluorophor als auch mit einem Quencher gekoppelt sind. Die Nucleotide am 5'-Ende der Sonde sind zu denen am 3'-Ende komplementär, so dass sich eine für Molecular Beacons charakteristische Sekundärstruktur ausbilden kann. In diesem als Haarnadelstruktur bezeichneten Zustand zeigt der Reporter durch seinen geringen Abstand zum Quencher keine Fluoreszenz. Durch Anlagerung der Schleifen-Region an die komplementäre DNA-Sequenz zwischen den Primern während eines PCR-Zyklus wird der Abstand zwischen Quencher und Reporter vergrößert. Die Reporter-Fluoreszenz kann somit beobachtet werden.

Eine weitere zur Durchführung des erfindungsgemäßen Verfahrens geeignete Methode ist die "Scorpion"-Technologie. Scorpion-Sonden sind komplexe Oligonucleotide, welche die Eigenschaften von Real-Time-PCR-Sonden und PCR-Primern in einem (Uni-Scorpion) oder zwei Molekülen (Bi-Scorpion) vereinigen. Ähnlich den "Molecular Beacons" besitzen sie eine charakteristische Sekundärstruktur mit einer selbstkomplementären Schaft-Region, deren Enden mit einem Reporter-Fluorophor und einem Quencher modifiziert wurden. Zusätzlich tragen diese Sonden am 3'-Ende einen PCR-Primer. Während eines PCR-Zyklus kann mit steigender DNA-Konzentration eine Reporter-Fluoreszenz durch Anlagerung der Loop-Region an eine komplementäre DNA-Sequenz und damit vergrößerten Abstand zwischen Quencher und Reporter beobachtet werden. Zum Nachweis des Bindens der unterschiedlichen Sonden werden diese mit fluoreszierenden Farbstoffmolekülen gekoppelt.

Weiterhin können bei der Durchführung des erfindungsgemäßen Verfahrens positive und/oder negative Kontrollgene, z.B., ein unmethyliertes Kontrollgen co-amplifziert werden.

Es ist bekannt, dass die Methylierung von Genen oft mit einer Transkriptionsblockade und dadurch einer fehlenden Translation in Zusammenhang steht. Somit umfasst das erfindungsgemäße Verfahren auch die indirekte Bestimmung des Methylierungsstatus durch Bestimmung der Konzentration der entsprechenden RNA bzw. des Proteins. Deren Nachweis kann durch übliche Verfahren erfolgen, z.B. (für RNA) über Northern-Analyse, RT-PCR etc. und (für Proteine) Antikörperbasierte Verfahren oder Verfahren, die auf der Bestimmung einer biologischen Aktivität des Proteins basieren.

Das erfindungsgemäße Verfahren kann beispielsweise auf den folgenden Schritten beruhen:
(a) Vom Zellabstrich der zu testenden Person wird DNA nach einem Standard-Verfahren isoliert, z. B. QiaAmp DNA-Mini Kit, nach Vorschrift des Herstellers (QIAGEN, Hilden, Deutschland);
(b) vorzugsweise wird in einem zweiten Schritt untersucht, ob die zu analysierende Probe hr-HPV-DNA enthält. Dieser Nachweis erfolgt mit einem bereits etablierten Verfahren wie z.B. dem GP5+/6+-PCR EIA-Verfahren (Jacobs et al., 1995, J Clin Microbiol 33:901-905). Sämtliche hr-HPV Typen (HPV16, 18, 26, 31, 33, 35, 39, 45, 51, 52, 53, 56, 58, 59, 66, 68, 73, 82) sollten erfasst werden. Durch den Nachweis eines house-keeping Gens, z.B. β-Globin oder β-Actin, wird sichergestellt, dass in der Probe eine ausreichende Menge an DNA von ausreichender Qualität vorliegt, die auch amplifizierbar ist;
(c) Bei einer HPV-negativen Probe ist das Vorliegen einer CIN2+ nahezu 100% ausgeschlossen (Cuzick et al., 2006; Int. J. Cancer. 119:1095-1101). Diese Proben müssen daher nicht weiter untersucht werden. Die Differenzierung zwischen Frauen mit einem hr-HPV-positiven Befund entweder mit oder ohne CIN2+ erfolgt dann über die Bestimmung des Methylierungsstatus der Gene ASTN1, DLX1, EDIL3, ITGA4, RXFP3 und ZNF671;
(d) Dazu wird die DNA der hr-HPV positiven Proben nach der Bisulfit-Methode, z. B. mittels eines kommerziellen Kits (z. B. Methylation Gold Kit, Zymo Research, Orange, CA, USA), chemisch konvertiert. Dabei werden durch Behandlung mit Natrium-Bisulfit und nachfolgende alkalische Hydrolyse alle nicht methylierten Cytosine der DNA-Probe in Uracile umgewandelt;
(e) Die relevante DNA wird mittels spezifischer Primer für die methylierte Form der jeweiligen Genomabschnitte durch PCR amplifiziert und analysiert;
(f) Zum Nachweis der Methylierung der Gene ASTN1, DLX1, EDIL3, ITGA4, RXFP3 und ZNF671 in einer methylierungsspezifischen PCR oder QMSP können die folgenden methylierungsspezifischen Primer eingesetzt werden:

| | |
|---|---|
| ASTN1_F | CGTAAGCGTTGTTAGCGTAGC |
| ASTN1_R | CGCGAAATCGAAACGAAAACG |
| DLX1_F | TATCGGGATTCGCGTTTGTAC |
| DLX1_R | CGACCGAACTAAAACTCAACTCG |
| EDIL3_F | GTTTTCGGCGGTTCGTTC |
| EDIL3_R | CGAACGCTCGACTATCGC |
| ITGA4_F | CGAATTCGGTTTTCGAAGGGTC |
| ITGA4_R | CACGACCGAATAACCGAACAAC |
| RXFP3_F | ATTTCGGAAAGCGTTTTTCGC |
| RXFP3_R | CTACGTCTCTCCGCGATTATC |
| ZNF671_F | CGGAGGACGTAGTATTTATTCGC |
| ZNF671_R | CTACGTCCCCGATCGAAACG |

Das erfindungsgemäße Diagnoseverfahren beschränkt sich aber nicht auf den Einsatz dieser Primer für der Nachweis der Methylierung der Gene ASTN1, DLX1, EDIL3, ITGA4, RXFP3 und ZNF671. Sie umfasst auch Primer anderer Sequenzen, die zum Nachweis der Methylierung der sechs Gene dienen können.
(g) Zum Nachweis der Methylierung der Gene ASTN1, DLX1, EDIL3, ITGA4, RXFP3 und ZNF671 durch MethyLight-Analyse werden zusätzlich zu den vorstehend beschriebenen Primern fluoreszierende Sonden eingesetzt:

| | |
|---|---|
| ASTN1 | GTAATTCGTTTGTTTCGTAAGTTGTTCG |
| DLX1 | CGTAAACGTTAGCTGTTCTGGAAACCG |
| EDIL3 | TCGTAGTCGTCGCGCGGAGAATA |
| ITGA4 | TTCGATCGGTCGTTTTTATAACG |
| RXFP3 | GCGTTTTGGGATTACGTATGTTTTTTGG |
| ZNF671 | CGTGGGCGCGGACAGTTGTCGGGAGCG |

Die Sonden werden, je nach Applikation, mit entsprechenden fluoreszenten Farbstoffen gekoppelt. Die Erfindung beschränkt sich aber nicht auf den Einsatz dieser Sonden für den Nachweis der Methylierung der Gene ASTN1, DLX1, EDIL3, ITGA4, RXFP3 und ZNF671. Sie umfasst auch Sonden mit anderen Sequenzen, die zum Nachweis der Methylierung der sechs Gene dienen können.

Eine Quantifizierung der Methylierung der Markergene ist nicht zwingend. Entscheidend ist, dass, z.B., durch MSP oder QMSP mindestens eine Zelle mit methylierten Markergen(en) in einem Hintergrund von 1000 Zellen ohne methylierte(s) Markergen(e) nachgewiesen werden kann.

In einer weiteren besonders bevorzugten Ausführungsform des erfindungsgemäßen Verfahrens wird zusätzlich die HPV-DNA in der Probe quantifiziert. Dies ermöglicht eine Abschätzung der Anzahl HPV-positiver Zellen in der durch Bisulfitbehandlung modifizierten DNA durch PCR mit entsprechend an die Bisulfit-Modifikation angepassten hr-HPV-spezifischen Oligonukleotidprimern. Die Primer erlauben vorzugsweise die Amplifikation in der konservierten L1-Region des HPV-Genoms und können beispielsweise die folgenden Sequenzen umfassen:

| | |
|---|---|
| HPV-F | GGTTATAATAATGGTATTTGTTGGG |
| HPV_R | TAAAAAATAAACTATAAATCATATTCC |

Ein weiterer Gegenstand der vorliegenden Erfindung ist ein Kit zur Durchführung eines erfindungsgemäßen Verfahrens. Ein solches Kit umfasst genspezifische Primer oder Primerpaare zur Bestimmung des Methylierungsstatus von ASTN1 (Astrotactin 1), DLX1 (distal-less homeobox 1) EDIL3 (EGF-like repeats and discoidin I-like domains 3), ITGA4 (α4-Integrin), RXFP3 (relaxin/insulin-like family peptide receptor 3), und ZNF671 (Zinkfingerprotein, Transkriptionsfaktor), vorzugsweise die vorstehend näher definierten Primer bzw. Primerpaare.

Das Kit kann zusätzlich auch eine Sonde wie vorstehend näher beschrieben und/oder ein Primerpaar für den Nachweis einer HPV-Infektion enthalten.

Schließlich kann das Kit in getrennten Behältern zusätzlich mindestens einen der folgenden Bestandteile enthalten:
(a)Reagenzien zur Isolierung von DNA;
(b)Enzyme zur DNA-Amplifikation;
(c)Natriumbisulfit; oder
(d)einen oder mehrere Puffer.

Von den einzelnen Komponenten des Kits können ein oder mehrere Vertreter vorliegen.

Mit der vorliegenden Erfindung ist es somit möglich, Karzinome des Anogenitaltrakts, insbesondere Zervixkarzinome, frühzeitig zu diagnostizieren. Insbesondere können Vorstufen dieser Karzinome frühzeitig erkannt werden. Zu betonen ist, dass zwischen hr-HPV positiven Frauen mit oder ohne klinisch relevante Gewebeveränderung, z.B. CIN2+, unterschieden werden kann. Kennzeichnend ist ferner, dass die durch ein erfindungsgemäßes Verfahren erzielten Ergebnisse nicht einer subjektiven Bewertung unterliegen, wodurch z.B. die falschnegativen bzw. falsch-positiven Ergebnisse eines Pap-Tests vermieden werden können. Des Weiteren zeichnet sich das vorliegende erfindungsgemäße Diagnoseverfahren durch schnelle und einfache Handhabung aus, wodurch es für große Screening-Maßnahmen, insbesondere auch in Ländern der Dritten Welt, geeignet ist. Somit liefert die vorliegende Erfindung einen wichtigen Beitrag zur modernen Diagnostik von Anogenitalkrebs, insbesondere des Zervixkarzinoms, oder Vorstufen davon.

Die Erfindung wird durch das folgende Beispiel erläutert.

### Beispiel 1

### DNA-Methylierung in Abstrichen

Untersucht wurden (1) hr-HPV positive Abstriche von 44 Frauen, bei denen es keinen Anhalt (zytomorphologisch und kolposkopisch) für eine Gewebeveränderung gab; (2) Abstriche von 49 hr-HPV positiven Frauen mit einer histopathologisch gesicherten CIN3 und (3) Abstriche von 65 hr-HPV positiven Frauen mit einem histopathologisch gesicherten Zervixkarzinom.

Die Ergebnisse sind in Figur 1 dargestellt (Methylierungshäufigkeit der Gene ASTN1, DLX1, EDIL3, ITGA4, RXFP3 und ZNF671 in den einzelnen Gruppen). Eine Methylierung mindestens eines der sechs Gene trat in 94% der CIN3- und 92% der Zervixkarzinomproben auf, hingegen nur in 11% der hr-HPV positiven Frauen ohne Gewebeveränderungen. Dies zeigt die Zuverlässigkeit des erfindungsgemäßen Verfahrens.

Die PCR für den Nachweis sowohl einer HPV-Infektion als auch für den Nachweis der methylierten Gene ist sehr empfindlich. Das vorliegende Beispiel beruht auf der jeweiligen qualitativen Bestimmung des HPV-Status und des Methylierungsstatus der Markergene. Durch eine quantitative Bestimmung der HPV DNA kann ein Grenzwert definiert werden, der einer Mindestanzahl HPV-positiver Zellen in der Probe entspricht. Dadurch kann die Spezifität der methylierungsspezifischen PCR für den Nachweis von CIN2+ weiter erhöht werden.

### Literatur

Cuzick et al., 2006; Int J Cancer 119:1095-1101
Esteller, 2007, Hum. Mol. Genet. 16: R50-R59
Jacobs et al., 1995, J Clin Mircobiol 33:901-905
Shames et al., 2007, Cancer Lett. 251:187-98
Yu et al., 2007, Clin Cancer Res 13: 7296 - 7304
Wentzensen et al., 2009; Gynecologic Oncology 112, 293-299

## Patentansprüche

1. Verfahren zum Nachweis von Anogenitalkrebs oder Vorstufen davon in einer Probe, umfassend die Bestimmung des Methylierungsstatus von ASTN1 (Astrotactin 1), DLX1 (distal-less homeobox 1), EDIL3 (EGF-like repeats and discoidin I-like domains 3), ITGA4 (α4-Integrin), RXFP3 (relaxin/insulin-like family peptide receptor 3) und/oder ZNF671 (Zinkfingerprotein, Transkriptionsfaktor).

2. Verfahren nach Anspruch 1, wobei der Anogenitalkrebs Gebärmutterhalskrebs ist.

3. Verfahren nach Anspruch 1 oder 2, wobei ASTN1, DLX1, EDIL3, ITGA4, RXFP3 und/oder ZNF671 in einer positiven Probe methyliert sind.

4. Verfahren nach Anspruch 2 oder 3, wobei die Probe ein Gebärmutterhalsabstrich ist.

5. Verfahren nach einem der Ansprüche 1 bis 4, wobei der Methylierungsstatus des Promotorbereichs bestimmt wird.

6. Verfahren nach einem der Ansprüche 1 bis 5, wobei der Methylierungsstatus des Gens (der Gene) mittels methylierungsspezifischer PCR (MSP) bestimmt wird.

7. Verfahren nach Anspruch 6, wobei die MSP eine quantitative MSP (QMSP) ist.

8. Verfahren nach Anspruch 6, wobei die quantitative MSP auf der MethyLight-Technik basiert.

9. Verfahren nach einem der Ansprüche 1 bis 6, wobei das Verfahren ein Multiplex-Verfahren ist.

10. Verfahren nach Anspruch 6, 7, 8 oder 9, wobei Primerpaare verwendet werden, die die folgende Sequenz umfassen:
(a) CGTAAGCGTTGTTAGCGTAGC (ASTN1_F) und CGCGAAATCGAAACGAAAACG (ASTN1_R);
(b) TATCGGGATTCGCGTTTGTAC (DLX1_F) und CGACCGAACTAAAACTCAACTCG (DLX1_R);
(c) GTTTTCGGCGGTTCGTTC (EDIL3_F) und CGAACGCTCGACTATCGC (EDIL3_R);
(d) CGAATTCGGTTTTCGAAGGGTC (ITGA4_F) und CACGACCGAATAACCGAACAAC (ITGA4_R);
(e) ATTTCGGAAAGCGTTTTTCGC (RXFP3_F) und CTACGTCTCTCCGCGATTATC (RXFP3_R); und/oder
(f) CGGAGGACGTAGTATTTATTCGC (ZNF671_F) und CTACGTCCCCGATCGAAACG (ZNF671_R).

11. Verfahren nach Anspruch 8 oder 9, wobei ein Primerpaar oder mehrere Primerpaare wie in Anspruch 10 definiert verwendet werden und zusätzlich mindestens eine Sonde, die eine der folgenden Sequenzen umfasst:
(a) GTAATTCGTTTGTTTCGTAAGTTGTTCG (ASTN1);
(b) CGTAAACGTTAGCTGTTCTGGAAACCG (DLX1)
(c) TCGTAGTCGTCGCGCGGAGAATA (EDIL3);
(d) TTCGATCGGTCGTTTTTATAACG (ITGA4);
(e) GCGTTTTGGGATTACGTATGTTTTTTGG (RXFP3); oder
(f) CGTGGGCGCGGACAGTTGTCGGGAGCG (ZNF671).

12. Verfahren nach einem der Ansprüche 1 bis 11, zusätzlich den Nachweis einer HPV-Infektion umfassend.

13. Verfahren nach Anspruch 12, wobei der HPV-Nachweis über PCR erfolgt.

14. Verfahren nach Anspruch 13, wobei ein Primerpaar verwendet wird, das folgende Sequenzen umfasst:
(a) GGTTATAATAATGGTATTTGTTGGG (HPV-F); und
(b) TAAAAAATAAACTATAAATCATATTCC (HPV_R).

15. Kit für die Verwendung zum Nachweis von Anogenitalkrebs oder Vorstufen davon in einer Probe, umfassend:
genspezifische Primer oder Primerpaare zur Bestimmung des Methylierungsstatus von ASTN1 (Astrotactin 1), DLX1 (distal-less homeobox 1), EDIL3 (EGF-like repeats and discoidin I-like domains 3), ITGA4 (α4-Integrin) RXFP3 (relaxin/insulin-like family peptide receptor 3) und/oder ZNF671 (Zinkfingerprotein, Transkriptionsfaktor)

16. Kit nach Anspruch 15, enthaltend die Primer/Primerpaare wie in Anspruch 10 definiert.

17. Kit nach Anspruch 15 bis 16, zusätzlich eine Sonde wie in Anspruch 11 definiert enthaltend.

18. Kit nach einem der Ansprüche 15 bis 17, zusätzlich ein Primerpaar für den Nachweis einer HPV-Infektion enthaltend.

19. Kit nach Anspruch 18, wobei das Primerpaar das in Anspruch 14 definierte Primerpaar ist.

20. Kit nach einem der Ansprüche 15 bis 19, der in getrennten Behältern zusätzlich mindestens einen der folgenden Bestandteile enthält:
(a) Reagenzien zur Isolierung von DNA;
(b) Enzyme zur DNA-Amplifikation;
(c) Natriumbisulfit; oder
(d) einen oder mehrere Puffer.
